(19) Europäisches Patentamt / European Patent Office / Office européen des brevets

(11) **EP 2 231 226 B1**

(12) **EUROPÄISCHE PATENTSCHRIFT**

(45) Veröffentlichungstag und Bekanntmachung des Hinweises auf die Patenterteilung:
**02.03.2016 Patentblatt 2016/09**

(21) Anmeldenummer: **08865010.6**

(22) Anmeldetag: **17.12.2008**

(51) Int Cl.:
*A61M 1/16* (2006.01)     *A61M 1/34* (2006.01)

(86) Internationale Anmeldenummer:
**PCT/EP2008/010727**

(87) Internationale Veröffentlichungsnummer:
**WO 2009/080258 (02.07.2009 Gazette 2009/27)**

(54) **VERFAHREN UND VORRICHTUNG ZUR BESTIMMUNG DES TRANSMEMBRANDRUCKS BEI EINER EXTRAKORPORALEN BLUTBEHANDLUNG**

METHOD AND DEVICE FOR DETERMINING THE TRANSMEMBRANE PRESSURE IN AN EXTRACORPOREAL BLOOD TREATMENT

PROCÉDÉ ET DISPOSITIF POUR DÉTERMINER LA PRESSION TRANSMEMBRANAIRE LORS D'UN TRAITEMENT EXTRACORPOREL DU SANG

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **22.12.2007 DE 102007062568**
**07.03.2008 DE 102008013089**

(43) Veröffentlichungstag der Anmeldung:
**29.09.2010 Patentblatt 2010/39**

(60) Teilanmeldung:
**16151137.3**

(73) Patentinhaber: **Fresenius Medical Care Deutschland GmbH**
**61352 Bad Homburg v.d.H. (DE)**

(72) Erfinder:
• **BALSCHAT, Klaus**
**97525 Schwebheim (DE)**
• **GAGEL, Alfred**
**96123 Litzendorf (DE)**
• **KÜLZ, Michael**
**66386 St. Ingbert (DE)**
• **SPICKERMANN, Reiner**
**97535 Wasserlosen-Burghausen (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**EP-A- 0 212 127       WO-A-03/028860**
**WO-A-2006/011009    US-A1- 2004 186 410**
**US-A1- 2004 217 056   US-B1- 6 526 357**

**Beschreibung**

Die Erfindung bezieht sich auf ein Verfahren zur Bestimmung des Transmembrandrucks während einer extrakorporalen Blutbehandlung, bei der Blut mit einer bestimmten Blutflussrate über eine arterielle Blutzuführleitung eines extrakorporalen Blutkreislaufs in den Einlass einer ersten Kammer eines durch eine semipermeable Membran in die erste Kammer und eine zweite Kammer unterteilten Dialysators strömt und über eine venöse Blutrückführleitung aus dem Auslass der ersten Kammer des Dialysators strömt, während Dialysierflüssigkeit über eine Dialysierflüssigkeitszuführleitung in den Einlass der zweiten Kammer des Dialysators strömt und über eine Dialysierflüssigkeitsabführleitung aus dem Auslass der zweiten Kammer des Dialysators strömt, wobei dem Blut mit einer bestimmten Flussrate über die Membran des Dialysators Flüssigkeit entzogen wird. Darüber hinaus betrifft die Erfindung eine extrakorporale Blutbehandlung, bei der der Transmembrandruck bestimmt wird. Des Weiteren betrifft die Erfindung eine Vorrichtung zur Bestimmung des Transmembrandrucks für eine Blutbehandlungsvorrichtung zur Durchführung einer extrakorporalen Blutbehandlung und eine extrakorporale Blutbehandlungsvorrichtung mit einer Vorrichtung zur Bestimmung des Transmembrandrucks.

Zur Entfernung von harnpflichtigen Substanzen und zum Flüssigkeitsentzug werden verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleineren molekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe, durch einen hohen Flüssigkeitsstrom (Konvektion) durch die Membran des Dialysators effektiv entfernt. Bei der Hämofiltration fungiert der Dialysator als Filter, der daher nachfolgend auch als Dialysator bezeichnet wird. Eine Kombination aus beiden Verfahren ist die Hämodiafiltration (HDF).

Bei der Hämo(dia)filtration (HDF) wird ein Teil des über die Membran des Dialysators entzogenen Serums durch eine sterile Substitutionsflüssigkeit ersetzt, die stromauf und/oder stromab des Dialysators dem extrakorporalen Blutkreislauf zugeführt wird. Die Zufuhr der Substitutionsflüssigkeit stromauf des Dialysators wird als Praedilution und die Zufuhr stromab des Dialysators als Postdilution bezeichnet.

Bei einer extrakorporalen Blutbehandlung ist die Ultrafiltrationsrate (UF-Rate) von Interesse, die ein Maß für die Menge der dem Patienten innerhalb eines Zeitintervalls entzogenen Flüssigkeit ist. Die Ultrafiltrationsrate ist bei der extrakorporalen Blutbehandlung von dem Transmembrandruck TMP abhängig, wobei die Ultrafiltrationsrate mit zunehmendem Transmembrandruck ansteigt.

Der Transmembrandruck TMP ist als der Druckunterschied zwischen dem mittleren, blutseitigen, und dem mittleren, dialysatseitigen Druck am Dialysator definiert. Für eine exakte Bestimmung des Transmembrandrucks sind grundsätzlich vier Druckmessungen erforderlich, wobei der Druck am Ein- und Auslass der Blutkammer und Ein- und Auslass der Dialysierflüssigkeitskammer des Dialysators gemessen wird. Hierzu ist jeweils ein Drucksensor am blutseitigen Ein- und Auslass und am dialysatseitigen Ein- und Auslass des Dialysators erforderlich.

In der Praxis erweist sich die Messung des Transmembrandrucks mittels vier Drucksensoren allerdings als relativ aufwendig. Aus Gründen der technischen Vereinfachung ist in der Praxis daher im Allgemeinen davon abgesehen worden, den Transmembrandruck mittels vier Drucksensoren zu bestimmen.

Es ist bekannt, zur Bestimmung des Transmembrandrucks den Druck nur mittels zwei Drucksensoren zu bestimmen, von denen der eine Drucksensor auf der Blutseite und der andere Drucksensor auf der Dialysatseite angeordnet ist. Aus Gründen der Handhabung und der Kosten wird beispielsweise in dem Artikel von H. D. Polaschegg "Methoden und Geschichte der Ultrafiltrationskontrolle in der Hämodialyse (Aktuelle Nephrologie, Heft 1/1985, S. 135 ff.) vorgeschlagen, sich auf die Messung des venösen Rücklaufdrucks sowie des Drucks am Dialysierflüssigkeitsauslauf zu beschränken.

Neben der Bestimmung des Transmembrandrucks mittels zwei Drucksensoren ist auch die Bestimmung des Transmembrandrucks mittels drei Drucksensoren bekannt. Die EP 0 212 127 beispielsweise schlägt vor, für die Bestimmung des Transmembrandrucks den Druck in der Dialysierflüssigkeitszuführ- und abführleitung und den Druck in der Blutrückführleitung, insbesondere der in der Blutrückführleitung angeordneten Tropfkammer, zu messen und den Transmembrandruck auf der Grundlage der gemessenen Drücke zu berechnen. Der berechnete Transmembrandruck wird mit einem voreingestellten Sollwert für den mittleren Transmembrandruck verglichen, um die in der Dialysierflüssigkeitsabführleitung angeordnete Dialysierflüssigkeitspumpe zu regeln. Die Saugpumpe auf der Dialysierflüssigkeitsseite wird derart geregelt, dass der Transmembrandruck im Dialysator auf dem Sollwert gehalten wird.

In der Praxis ist die Bestimmung des Transmembrandrucks auf der Grundlage von nur zwei oder drei Druckmessungen, von denen jeweils eine Druckmessung auf der Blut- und die andere Messung auf der Dialysatseite erfolgt, als ausreichend genau angesehen worden. Die Erfinder haben jedoch erkannt, dass unter gewissen Behandlungsbedingungen der Bestimmung des Transmembrandrucks mit hoher Genauigkeit Grenzen gesetzt sind.

Die WO 03/028860 A1 beschreibt eine Vorrichtung zur extrakoporalen Blutbehandlung, die über eine Einrichtung zur Bestimmung des Transmembrandrucks verfügt. Der Transmembrandruck wird nur auf der Grundlage einer Druckmessung auf der Blutseite stromab des Dialysators und der Dialysierflüssigkeitsseite stromab des Dialysators überwacht. Aus dieser Druckschrift geht hervor, dass die Eigenschaften der Membran des Dialysators und die Zusammensetzung des Bluts den Transmembrandruck beeinflussen. Aus der US 2004/0186410 A1 ist bekannt, dass der Transmembrandruck vom Hämatokrit abhängig ist.

Der Erfindung liegt die Aufgabe zu Grunde, ein Verfahren zur Bestimmung des Transmembrandrucks bei einer extrakorporalen Blutbehandlung anzugeben, das einerseits einen nur verhältnismäßig geringen technischen Aufwand für die Messung erforderlich macht und andererseits eine hohe Genauigkeit unter allen Behandlungsbedingungen gewährleistet.

Darüber hinaus ist es eine Aufgabe der Erfindung eine Vorrichtung zur Bestimmung des Transmembrandrucks für eine extrakorporale Blutbehandlungsvorrichtung bereitzustellen, die mit weniger als vier Drucksensoren unter allen Behandlungsbedingungen eine Bestimmung des Transmembrandrucks mit hoher Genauigkeit erlaubt.

Eine weitere Aufgabe der Erfindung ist eine extrakorporale Blutbehandlungsvorrichtung anzugeben, wobei mit hoher Genauigkeit die Bestimmung des Transmembrandrucks mit verhältnismäßig geringem technischen Aufwand erfolgt.

Die Lösung dieser Aufgaben erfolgt erfindungsgemäß mit den Merkmalen der Patentansprüche 1, 9 und 17. Vorteilhafte Ausführungsformen der Erfindung sind Gegenstand der Unteransprüche.

Das erfindungsgemäße Verfahren und die erfindungsgemäße Vorrichtung zur Bestimmung des Transmembrandrucks beruhen darauf, dass mit einem verhältnismäßig geringen technischen Aufwand der Druck auf der Blut- und Dialysierflüssigkeitsseite des Dialysators mit weniger als vier Drucksensoren gemessen und ein vorläufiger unkorrigierter Wert für den Transmembrandruck berechnet wird, der anschließend mit einer Korrekturgröße korrigiert wird, die von einer mit der Viskosität des Bluts korrelierenden Größe abhängig ist. Folglich findet bei der Bestimmung des Transmembrandrucks eine mit der Viskosität des Bluts korrelierende Größe, insbesondere der Hämatokrit des Bluts Berücksichtigung.

Die Erfinder haben erkannt, dass es insbesondere bei einer starken Eindickung oder Verdünnung des Bluts, wie sie bei der Hämodiafiltrationsbehandlung oder der Hämofiltrationsbehandlung auftreten kann, zu Abweichungen zwischen dem tatsächlichen Transmembrandruck und dem Wert für den Transmembrandruck kommen kann, der sich aus der Messung des Drucks an weniger als vier Messstellen ergibt, beispielsweise bei einer Messung des Drucks nur am Ein- oder Auslass, nicht aber am Ein- und Auslass der jeweiligen Kammer des Dialysators.

Bei der Korrekturgröße für den Transmembrandruck handelt es sich um eine für den Strömungswiderstand des Dialysators in Längsrichtung charakteristische Kenngröße, die wiederum von einer mit der Viskosität des Bluts korrelierenden Kenngröße, insbesondere von dem Hämatokrit abhängig ist.

Die Bestimmung des Transmembrandrucks ist mit dem erfindungsgemäßen Verfahren auch dann besonders genau, wenn dem Patienten Erythropoietin (EPO) verabreicht wird, wodurch sich der Hämatokrit vergrößert und die Viskosität des Bluts zunimmt.

Bei einer bevorzugten Ausführungsform des erfindungsgemäßen Verfahrens bzw. der erfindungsgemäßen Vorrichtung wird der Druck auf der Blutseite in der Blutrückführleitung am Auslass der ersten Kammer des Dialysators gemessen, während auf der Dialysierflüssigkeitsseite der Druck in der Dialysierflüssigkeitszuführleitung am Einlass der zweiten Kammer und in der Dialysierflüssigkeitsabführleitung am Auslass der zweiten Kammer des Dialysators gemessen wird. Damit ist es nicht erforderlich, den Druck auf der Blutseite in der Blutzuführleitung am Einlass der ersten Kammer des Dialysators zu messen, so dass die Druckmessung mit nur drei Drucksensoren erfolgen kann.

Es ist aber auch möglich, dass auf der Blutseite der Druck nicht am Auslass, sondern am Einlass der ersten Kammer des Dialysators gemessen wird. Ebenfalls ist es möglich, dass der Druck auf der Blutseite sowohl am Ein- und Auslass der ersten Kammer des Dialysators gemessen wird, während der Druck auf der Dialysierflüssigkeitsseite nur entweder am Einlass oder am Auslass der zweiten Kammer des Dialysators gemessen wird. Entscheidend ist, dass zumindest eine Druckmessung sowohl auf der Blutseite als auch der Dialysierflüssigkeitsseite des Dialysators erfolgt.

Wenn von einer Messung des Drucks am Ein- oder Auslass einer der beiden Kammern des Dialysators die Rede ist, muss darunter nicht zwingend verstanden werden, dass die Messung direkt an dem Punkt erfolgen muss, an dem die Leitungen an dem Dialysator angeschlossen ist. Vielmehr ist es auch möglich, die Messung stromauf bxw. stromab des Ein- bzw. Auslasses vorzunehmen, wobei davon auszugehen ist, dass der Druckanstieg bzw. Druckabfall zwischen dem eigentlichen Messpunkt und dem Eingang bzw. Ausgang der jeweiligen Kammer des Dialysators gering ist.

Es hat sich gezeigt, dass die Abweichungen zwischen dem Transmembrandruck, der auf der Grundlage einer Messung mit weniger als vier Drucksensoren berechnet wird, und dem tatsächlichen Transmembrandruck mit zunehmendem Strömungswiderstand des Dialysators in Längsrichtung zunimmt. Da bei der Bestimmung des Transmembrandrucks nach dem erfindungsgemäßen Verfahren und der erfindungsgemäßen Vorrichtung der Strömungswiderstand des Dialysators in Längsrichtung Berücksichtigung findet, kann der tatsächliche Transmembrandruck mit hoher Genauigkeit berechnet werden.

Der Strömungswiderstand des Dialysators in Längsrichtung, der von einer mit der Viskosität des Bluts korrelierenden Kenngröße, insbesondere dem Hämatokrit abhängig ist, kann grundsätzlich zu Beginn der Blutbehandlung oder während der Blutbehandlung berechnet werden.

Eine besonders bevorzugte Ausführungsform der Erfindung sieht eine fortlaufende Bestimmung der mit der Viskosität des Bluts korrelierenden Kenngröße, insbesondere des Hämatokrits, während der Blutbehandlung vor, wobei der Hämatokrit online gemessen wird.

Die Abhängigkeit des Längswiderstands des Dialysators von der mit der Viskosität des Bluts korrelierenden Größe, insbesondere von dem Hämatokrit, wird vorzugsweise durch einen Polynom-Ansatz beschrieben werden, dessen Parameter aus individuellen Messdaten für jeden relevanten Dialysatortyp unter der Annahme einer Prae- oder Postdilution bestimmt werden.

Die Erfinder haben erkannt, dass der Strömungswiderstand in Längsrichtung des Dialysators im Wesentlichen von der Bauform des Dialysators, der sich durch eine bestimmte Membranfläche oder -länge und einen bestimmten Durchmesser der Kapillaren auszeichnet, von der Behandlungsart, beispielsweise einer HD-Behandlung oder H(D)F-Behandlung mit Praedilution oder Postdilution, von der Substitutionsrate und von der Ultrafiltrationsrate und den Blutinhaltsstoffen abhängt. Bei einer bevorzugten Ausführungsform der Erfindung finden daher die obigen Größen bei dem Polynom-Ansatz zur Bestimmung des Längswiderstands des Dialysators Berücksichtigung.

Die Korrekturgröße für den Transmembrandruck wird vorzugsweise auf der Grundlage des Produkts der für den Strömungswiderstand in Längsrichtung des Dialysators charakteristischen Kenngröße und der Blutflussrate im extrakorporalen Blutkreislauf bestimmt. Daher ist die Korrekturgröße auch von dem Blutfluss abhängig.

Das erfindungsgemäße Verfahren und die erfindungsgemäße Vorrichtung gehen also davon aus, dass die Abweichungen zwischen dem auf der Grundlage der gemessenen Drücke berechneten Transmembrandruck und dem tatsächlichen Transmembrandruck mit zunehmender Viskosität des Bluts und mit zunehmendem Blutfluss zunehmen.

Die erfindungsgemäße Vorrichtung zur Bestimmung des Transmembrandrucks, die für eine Blutbehandlungsvorrichtung zur Durchführung einer extrakorporalen Blutbehandlung bestimmt ist, verfügt über Mittel zum Messen des Drucks auf der Blut- und der Dialysierflüssigkeitsseite und Mittel zum Berechnen des Transmembrandrucks unter Berücksichtigung der Korrekturgröße. Die Mittel zum Messen des Drucks auf der Blut- und der Dialysierflüssigkeitsseite des Dialysators umfassen bei einer bevorzugten Ausführungsform Mittel zum Messen des Drucks in der Blutrückführleitung am Auslass der ersten Kammer des Dialysators sowie Mittel zum Messen des Drucks in der Dialysierflüssigkeitszuführ- und abführleitung am Einlass bzw. Auslass der zweiten Kammer des Dialysators. Mittel zum Messen des Drucks in der Blutzuführleitung am Einlass der ersten Kammer des Dialysators sind somit nicht erforderlich.

Die Mittel zum Messen des Drucks können konventionelle Drucksensoren sein, die bei den bekannten Blutbehandlungsvorrichtungen ohnehin vorhanden sind. Die Mittel zum Berechnen des Transmembrandrucks können ein konventioneller Mikroprozessor oder dergleichen sein, der ebenfalls ohnehin in den bekannten Blutbehandlungsvorrichtungen vorhanden ist.

Im Folgenden wird ein Ausführungsbeispiel des erfindungsgemäßen Verfahrens sowie der erfindungsgemäßen Vorrichtung unter Bezugnahme auf die anliegende Figur im Einzelnen beschrieben.

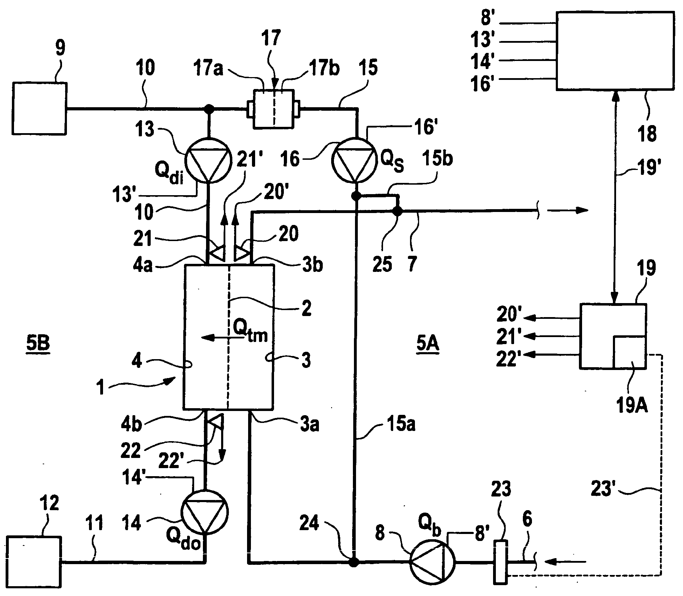

Die Figur zeigt nur die wesentlichen Komponenten einer Blutbehandlungsvorrichtung für eine extrakorporale Blutbehandlung zusammen mit einer Vorrichtung zur Bestimmung des Transmembrandrucks in stark vereinfachter schematischer Darstellung.

Die erfindungsgemäße Vorrichtung zur Messung des Transmembrandrucks kann Bestandteil einer konventionellen Blutbehandlungsvorrichtung sein oder eine separate Geräteeinheit sein, die mit der Blutbehandlungsvorrichtung zusammenwirkt.

Bei der vorliegenden Blutbehandlungsvorrichtung handelt es sich um eine Hämo(dia)filtrationsvorrichtung, die einen Dialysator 1 aufweist, der durch eine semipermeable Membran 2 in eine von Blut durchflossene erste Kammer 3, die nachfolgend als Blutkammer bezeichnet wird, und eine von Dialysierflüssigkeit durchflossene zweite Kammer 4 getrennt ist, die als Dialysierflüssigkeitskammer bezeichnet wird. Die erste Kammer 3 ist in einen extrakorporalen Blutkreislauf 5A geschaltet, während die zweite Kammer 4 in das Dialysierflüssigkeitssystem 5B der Hämo(dia)filtrationsvorrichtung geschaltet ist.

Der extrakorporale Blutkreislauf 5A umfasst eine arterielle Blutzuführleitung 6, die zu dem Einlass 3a der Blutkammer 3 führt, und eine venöse Blutrückführleitung 7, die von dem Auslass 3b der Blutkammer 3 des Dialysators 1 abgeht. Das Blut des Patienten wird durch die Blutkammer 3 des Dialysators 1 mit einer arteriellen Blutpumpe 8, insbesondere Rollenpumpe gefördert, die an der arteriellen Blutrückführleitung 6 angeordnet ist. Die Blutpumpe führt der Blutkammer 3 des Dialysators Blut mit einer bestimmten Blutflussrate $Q_b$ zu. Zur Eliminierung von Luftblasen kann in die arterielle und venöse Blutleitung ein Luftabscheider (Tropfkammer) geschaltet sein.

Bei den Blutleitungen 6, 7 der Blutbehandlungsvorrichtung handelt es sich um Schlauchleitungen, die in die Rollenpumpen zur einmaligen Verwendung eingelegt werden. Daher sind die Schlauchleitungen grundsätzlich nicht Bestandteil der Blutbehandlungsvorrichtung. Auch der Dialysator ist grundsätzlich nicht Bestandteil der Blutbehandlungsvorrichtung, sondern wird zur einmaligen Verwendung an die Schlauchleitungen angeschlossen.

Die frische Dialysierflüssigkeit wird in einer Dialysierflüssigkeitsquelle 9 bereitgestellt. Von der Dialysierflüssigkeitsquelle 9 führt eine Dialysierflüssigkeitszuführleitung 10 zu dem Einlass 4a der Dialysierflüssigkeitskammer 4 des Dialysators 1. Von dem Auslass 4b der Dialysierflüssigkeitskammer 4 führt eine Dialysierflüssigkeitsabführleitung 11 zu einem Abfluss 12. In die Dialysierflüssigkeitszuführleitung 10 ist eine erste Dialysierflüssigkeitspumpe 13 und in die Dialysierflüssigkeitsabführleitung 11 ist eine zweite Dialysierflüssigkeitspumpe 14 geschaltet. Die erste Dialysierflüssigkeitspumpe 13 fördert Dialysierflüssigkeit von der Dialysierflüssigkeitsquelle mit einer bestimmten Dialysierflüssigkeitszuführrate $Q_{di}$ zu dem Einlass 4a der Dialysierflüssigkeitskammer 4, während die zweite Dialysierflüssigkeitspumpe 14 von dem Auslass 4b der Dialysierflüssigkeitskammer 4 Dialysierflüssigkeit mit einer bestimmten Dialysierflüssigkeitsabführrate $Q_{do}$ zu dem Abfluss 12 fördert.

Während der Dialysebehandlung kann Dialysierflüssigkeit aus dem Dialysierflüssigkeitssystem 5B als Substitutionsflüssigkeit über eine Substitutionsflüssigkeitsleitung 15 dem extrakorporalen Blutkreislauf 5A zugeführt werden, die stromauf der ersten Dialysierflüssigkeitspumpe 13 von der Dialysierflüssigkeitszuführleitung 10 abzweigt.

Die Substitutionsflüssigkeitsleitung 15 weist zwei Leitungsabschnitte 15a und 15b auf, von denen der eine Leitungsabschnitt 15a zu der arteriellen Blutleitung 6 und der andere Leitungsabschnitt 15b zu der venösen Blutleitung 7 führt.

Die Substitutionsflüssigkeit wird mittels einer Substituatpumpe 16, insbesondere Rollenpumpe gefördert, in die die Substitutionsflüssigkeitsleitung 15 eingelegt ist. In die Substitutionsflüssigkeitsleitung 15 ist stromauf der Substituatpumpe eine in zwei Kammern 17a, 17b unterteiltes Sterilfilter 17 geschaltet. Die Substituatpumpe zusammen mit den zugehörigen Leitungen und dem Sterilfilter bilden die Substitutionseinrichtung der Dialysevorrichtung. Zum Abklemmen der beiden Leitungsabschnitte 15a, 15b der Substitutionsflüssigkeitsleitung 15 können Absperrorgane, beispielsweise Schlauchklemmen vorgesehen sein, die aber der besseren Übersichtlichkeit halber nicht dargestellt sind.

Die Blutpumpe 8, die erste und zweite Dialysierflüssigkeitspumpe 13 und 14 sowie die Substituatpumpe 16 sind über Steuerleitungen 8', 13', 14' 16' mit einer zentralen Steuer- und Recheneinheit 18 verbunden, von der die Pumpen unter Berücksichtigung der vorgegebenen Behandlungsparameter angesteuert werden. Die Steuer- und Recheneinheit 18 steuert auch die nicht dargestellten Absperrorgane an, um eine Blutbehandlung mit Prae- oder Postdilution durchzuführen.

Zum Betrieb der Hämo(dia)filtrationsvorrichtung als Hämodialysevorrichtung werden die Blutpumpe 8 sowie die erste und zweite Dialysierflüssigkeitspumpe 13 und 14 betrieben, wobei Dialysierflüssigkeit durch die Dialysierflüssigkeitskammer 4 des Dialysators 1 strömt. Zum Betrieb der Hämo(dia)filtrationsvorrichtung als Hämodiafiltrationsvorrichtung wird die Substituatpumpe 16 betrieben, so dass über den Sterilfilter 17 sterile Dialysierflüssigkeit als Substitutionsflüssigkeit wahlweise zu der arteriellen Zugabestelle 24 stromab der Blutpumpe 8 und stromauf der Blutkammer 3 (Praedilution) oder zu der venösen Zugabestelle 25 stromab der Blutkammer (Postdilution) strömt. Es ist aber grundsätzlich auch ein Betrieb der Hämo(dia)filtrationsvorrichtung nur als Hämofiltrationsvorrichtung möglich, wenn die erste Dialysierflüssigkeitspumpe 13 nicht betrieben wird und somit der Zufluss von Dialysierflüssigkeit in die Dialysierflüssigkeitskammer des Dialysators unterbrochen wird.

In der zentralen Steuer- und Recheneinheit 18 der Blutbehandlungsvorrichtung findet die Verarbeitung der die für die Blutbehandlung charakteristischen Behandlungsparameter statt. Diese charakteristischen Größen können entweder vom Bediener der Maschine vorgegeben, während der Behandlung gemessen und/oder aus gemessenen und/oder vorgegebenen Größen berechnet werden. Im Folgenden wird davon ausgegangen, dass sämtliche der hier relevanten Größe von der zentralen Steuer- und Recheneinheit bereitgestellt werden, da sie vom Bediener über eine nicht dargestellte Tastatur eingegeben und/oder mit nicht dargestellten Messeinheiten gemessen und/oder aus den eingegebenen und/oder gemessenen Größen berechnet werden.

Die erfindungsgemäße Vorrichtung zur Bestimmung des Transmembrandrucks kann eine selbstständige Baugruppe bilden oder Bestandteil der zentralen Steuer- und Recheneinheit 18 der Blutbehandlungsvorrichtung sein. Im vorliegenden Ausführungsbeispiel bilden die relevanten Komponenten der Vorrichtung zur Bestimmung des Transmembrandrucks eine separate Baugruppe, die nachfolgend im Einzelnen beschrieben wird.

Die Vorrichtung zur Bestimmung des Transmembrandrucks verfügt über eine zentrale Recheneinheit 19, beispielsweise einen Mikroprozessor, bei dem es sich auch um den Mikroprozessor handeln kann, der in der zentralen Steuer- und Recheneinheit 18 der Blutbehandlungsvorrichtung vorgesehen ist. Darüber hinaus verfügt die Vorrichtung zum Bestimmen des Transmembrandrucks über insgesamt drei Drucksensoren 20, 21, 22, von denen der erste Drucksensor den Druck am Auslass 3b der ersten Kammer 3 des Dialysators 1, der zweite Drucksensor 21 den Druck am Einlass 4a der zweiten Kammer 4. und der dritte Drucksensor 22 den Druck am Auslass 4b der zweiten Kammer 4 des Dialysators 1 misst. Diese Drucksensoren müssen nicht direkt am Einlass bzw. Auslass des Dialysator angeordnet sein. Entscheidend ist, dass mit hinreichender Genauigkeit der Druck am blutseitigen Ausgang und am dialysatseitigen Ein- und Ausgang des Dialysators gemessen wird.

Die Recheneinheit 19 empfängt die Messwerte der Drucksensoren 20, 21, 22 über Datenleitungen 20', 21', und 22'. Darüber hinaus kommuniziert die Recheneinheit 19 über eine weitere Datenleitung 19' mit der zentralen Steuer- und Recheneinheit 18 der Blutbehandlungsvorrichtung, um die hier relevanten Größen zu empfangen, die vom Bediener eingegeben und/oder von nicht dargestellten Sensoren gemessen und/oder berechnet werden.

Bei einer bevorzugten Ausführungsform verfügt die Vorrichtung zum Bestimmen des Transmembrandrucks noch über eine Messeinheit 23 zum Messen des Hämatokrits des im extrakorporalen Blutkreislaufs 5A strömenden Bluts, der sich im Laufe der extrakorporalen Blutbehandlung ändern kann. Aufgrund der Ultrafiltration steigt der Hämatokrit während der Blutlaufbehandlung im Allgemeinen an. Die Recheneinheit 19 ist mit der Messeinheit 23 zur Bestimmung des Hämatokrit über eine Datenleitung 23' verbunden. Messeinheiten zur Bestimmung des Hämatokrits sind dem Fachmann aus dem Stand der Technik bekannt.

Nachfolgend werden die theoretischen Grundlagen zur Bestimmung des Transmembrandrucks sowie die erfindungsgemäße Vorrichtung zur Bestimmung des Transmembrandrucks und das erfindungsgemäße Verfahren, nach dem die Vorrichtung zur Bestimmung des Transmembrandrucks arbeitet, im Einzelnen beschrieben.

Zur exakten Bestimmung des mittleren Transmembrandrucks TMP sind grundsätzlich vier Drucksensoren notwendig. Nach der Messung des Drucks am blutseitigen Einlass $P_{b,in}$, des Drucks am blutseitigen Auslass $P_{b,out}$, des Drucks am dialysatseitigen Einlass $P_{d,in}$ und des Drucks am dialysatseitigen Auslass $P_{d,out}$ kann der Transmembrandruck $P_{TM}$ (TMP) nach der folgenden Gleichung berechnet werden

$$\text{TMP} = P_{TM} = \frac{P_{b,in} + P_{b,out}}{2} - \frac{P_{d,in} + P_{d,out}}{2} \tag{1}$$

mit

$P_{TM}$     Transmembrandruck TMP
$P_{b,in}$     Druck am blutseitigen Einlass des Dialysators
$P_{b,out}$     Druck am blutseitigen Auslass des Dialysators (= venöser Druck $P_{ven}$)
$P_{d,in}$     Druck am dialysatseitigen Einlass des Dialysators
$P_{d,out}$     Druck am dialysatseitigen Auslass des Dialysators

Bei dem vorliegenden Ausführungsbeispiel wird der Druck aber nicht mittels vier Drucksensoren an den oben angegebenen Messstellen, sondern nur mittels drei Drucksensoren 20, 21, 22 gemessen, die den Druck $P_{b,out}$ am blutseitigen Auslass 3b der Blutkammer 3 des Dialysators 1, den Druck $P_{d,in}$ am dialysatseitigen Einlass 4a und den Druck $P_{d,out}$ am dialysatseitigen Auslass 4b der Dialysierflüssigkeitskammer 4 des Dialysators 1 messen.

Die Unterschiede zwischen der Bestimmung des Transmembrandrucks auf der Grundlage von einer Messung an drei Messstellen und einer Messung an vier Messstellen resultieren aus dem Druckabfall $\Delta P_b$ auf der Blutseite des Dialysators, der mit zunehmender Viskosität des Bluts, zunehmendem Blutfluss $Q_b$ und kleinerem Kapillardurchmesser bei gleicher Membranfläche ansteigt. Entsprechend der möglichen Kombinationen der Randbedingungen können sich kleinere oder größere Differenzen zwischen den beiden Messungen ergeben.

Darüber hinaus kann durch das Behandlungsverfahren die Viskosität des Bluts im Dialysator verändert werden. Bei einer H(D)F-Behandlung verringert sich beispielsweise die mittlere Blutviskosität im Dialysator (Filter) bei Praedilution, während sich die mittlere Blutviskosität bei Postdilution erhöht. Daher führt die Postdilution zu größeren Unterschieden bei den beiden Messungen. Dies ist auf den unterschiedlichen Transmembranfluss über die Membran des Dialysators zurückzuführen, der dem Blutstrom $Q_b$ entzogen wird. Der gesamte Transmembranfluss $Q_{tm} = Q_{uf} + Q_{sub}$ setzt sich aus der Ultrafiltrationsrate $Q_{uf}$ und der Substitutionsrate $Q_{sub}$ zusammen. In der Praxis kann jedoch die Substitutionsrate $Q_{uf}$ häufig vernachlässigt werden.

Die Erfindung beruht darauf, den Transmembrandruck $P_{TM3}$ auf der Grundlage des mit den drei Drucksensoren 20, 21, 22 gemessenen Drucks zu berechnen und eine Korrekturgröße für den berechneten Transmembrandruck zu bestimmen, um den tatsächlichen Transmembrandruck $P_{TM}$ = TMP zu ermitteln.

Durch Umformung der Gleichung (1) ergibt sich:

$$P_{TM} = P_{b.out} - \frac{P_{d,in} + P_{d,out}}{2} + \frac{P_{b,in} - P_{b,out}}{2} \tag{2}$$

Darin ist der unkorrigierte Transmembrandruck $P_{TM3}$ enthalten:

$$P_{TM3} = P_{b,out} - \frac{P_{d,in} + P_{d,out}}{2} \qquad (3)$$

Der Korrekturterm ergibt sich durch Vergleich von Gleichung (3) und Gleichung (2) aus dem letzten Glied von Gleichung (2). Er spiegelt den blutseitigen Druckabfall auf der Längsseite der Blutkammer 3 des Dialysators 1 wieder:

$$\frac{P_{b,in} - P_{b,out}}{2} = \frac{\Delta P_b}{2} \qquad (4)$$

mit: $\Delta P_b$ Druckabfall auf der Längsseite des Dialysators (blutseitig)

Der Druckabfall auf der Blutseite des Dialysators hängt im Wesentlichen vom Blutfluss $Q_b$ ab. Durch einen Polynom-Ansatz

$$\Delta P_b = \sum_{i=0}^{n} c_i * Q_b^i \qquad (5)$$

kann dieser Zusammenhang allgemein beschrieben werden. In der Praxis ergeben sich in der Regel mit hinreichender Genauigkeit lineare Abhängigkeiten zwischen dem Druckabfall $\Delta P_b$ und dem Blutfluss $Q_b$. Der Druckabfall auf der Blutseite $\Delta P_b$ kann damit in einen Strömungswiderstand $R_b$ in Längsrichtung des Dialysators, der vom Blutfluss $Q_b$ unabhängig ist, und den aktuellen Blutfluss $Q_b$ aufgeteilt werden. Damit ergibt sich:

$$P_{TM} = P_{TM3} + \frac{1}{2} * R_b * Q_b \qquad (6)$$

mit:

$R_b$    Längswiderstand des Dialysators auf der Blutseite
$Q_b$    Blutfluss

Bei dem vorliegenden Ausführungsbeispiel wird zur Berechnung des Strömungswiderstands $R_b$ in Längsrichtung der Blutkammer 3 des Dialysators 1 ein Polynom-Ansatz mit den Parametern $\alpha_0$, $\alpha_1$, $\alpha_2$, $\alpha_3$, $\alpha_4$ ... verwendet. Ein Beispiel für einen möglichen Polynom-Ansatz ist:

$$R_b(Hkt, Q_{tm}) = a_0 + a_1 * Hkt + a_2 * \frac{Q_{tm}}{Q_{tm,max}} + a_3 * Hkt * \frac{Q_{tm}}{Q_{tm,max}} + a_4 * \left( Hkt * \frac{Q_{tm}}{Q_{tm,max}} \right)^4 \qquad (7)$$

Dabei können $Q_{tm,max}$ für den Fall der Postdilution oder der Praediltution wie folgt bestimmt werden:

$$Q_{tm,Post,max} = Q_b * (1 - Hkt) * \left( 1 - k * \frac{TP}{100} \right) \qquad (8)$$

oder:

$$Q_{tm,Prae,max} = Q_{tm,Post,max} \left( k * \frac{TP}{100 g / dl} \right) \qquad (9)$$

wobei k ein Faktor ist, beispielsweise *k*=7, und wobei:

H*kt*    Hämatokrit [0,10..0,69]
TP    Totaler Proteingehalt [5,0...9,0 g/dl]
$Q_{tm}$    Aktuelle Flussrate über die Dialysator-Membran [ml/min]; mit: $Q_{tm} = Q_{sub} + Q_{uf}$
$Q_{sub}$    Substitutionsrate [ml/min];
$Q_{uf}$    Ultrafiltrationsrate [ml/min];
$Q_{tm,max}$    Maximale Flussrate [ml/min] bei

•Postdilution: Qtm,Post,max nach Gl. (8), oder bei
•Praedilution: $Q_{tm,Prae,max}$ nach Gl. (9).

Anstelle des Polynom-Ansatzes nach Gleichung (7) ist auch ein allgemeinerer Ansatz möglich, der höhere Potenzen für den Hämatokrit H*kt*, für den Transmembranfluss $Q_{tm}$ und dem Produkt aus Hämatokrit und Transmembranfluss berücksichtigt.

$$R_b\left(Hkt, Q_{tm}\right) = \sum_{i=0}^{n} b_{1,i} * Hkt^i + \sum_{j=1}^{m} b_{2,j} * \left(\frac{Q_{tm}}{Q_{tm,max}}\right)^j + \sum_{k=1}^{p} b_{3,k} * \left(Hkt * \frac{Q_{tm}}{Q_{tm,max}}\right)^k$$

$$(10)$$

Die erfindungsgemäße Vorrichtung bestimmt den Transmembrandruck TMP wie folgt.

Die Recheneinheit 19 der Vorrichtung zur Bestimmung des Hämatokrits berechnet zunächst nach Gleichung (7) den Längswiderstand $R_b$ des Dialysators als Funktion des Hämatokrit H*kt* und der Flussrate $Q_{tm}$ der über die Membran 2 des Dialysators 1 entzogenen Flüssigkeit. Hierzu greift die Recheneinheit auf einen Speicher 19A zu, in dem die Parameter des Polynom-Ansatzes $\alpha_0, \alpha_1, \alpha_2, \alpha_3, \alpha_4$ gespeichert sind, die durch eine Ausgleichsrechnung aus individuellen Messdaten für einen bestimmten Dialysatortyp gewonnen worden sind. In dem Speicher 19A der Recheneinheit 19 können die Parameter für verschiedene Dialysatortypen abgelegt werden, wobei die Recheneinheit dann auf die für den derzeit verwendeten Dialysatortyp zutreffenden Parameter zugreift.

Die Recheneinheit 19 kommuniziert mit der zentralen Steuer- und Recheneinheit 18 der Blutbehandlungsvorrichtung, um die hier relevanten Daten auszutauschen. Beispielsweise kann die Recheneinheit einen Datensatz empfangen, der den Dialysatortyp angibt, der zuvor vom Bediener beispielsweise mittels einer Tastatur eingegeben worden ist. Darüber hinaus empfängt die Recheneinheit 19 von der zentralen Steuer- und Recheneinheit 18 die Substitutionsrate $Q_{sub}$ und die Ultrafiltrationsrate $Q_{uf}$ ,um aus der Summe der Substitutionsrate und der Ultrafiltrationsrate die Flussrate $Q_{tm} = Q_{sub} + Q_{uf}$ der über die Membran 2 des Dialysators 1 entzogenen Flüssigkeit zu berechnen. Des Weiteren empfängt die Recheneinheit 19 von der zentralen Steuer- und Recheneinheit 18 den Hämatokrit H*kt*, der zwischen 0,10 und 0,69 liegen kann, und den totalen Proteingehalt TP, der zwischen 5,0 und 9,0 g/dl liegen kann. Weiterhin empfängt die Recheneinheit von der zentralen Steuer- und Recheneinheit ein Signal, das angibt, ob eine Prae- oder Postdilution vorliegt.

Nach den Gleichungen (8) und (9) berechnet die Recheneinheit 19 aus dem Hämatokrit H*kt* und dem totalen Proteingehalt TP die maximale Flussrate $Q_{tm,max}$ für den Fall, dass eine Prae- bzw. eine Postdilution vorgenommen wird.

Bei einer vereinfachten Ausführungsform wird der Längswiderstand $R_b$ des Dialysators nur einmal vor oder während der Dialysebehandlung berechnet. Eine verbesserte Ausführungsform sieht jedoch vor, dass der Längswiderstand $R_b$ des Dialysators zu bestimmten Zeitpunkten der Blutbehandlung mehrmals berechnet oder sogar während der Blutbehandlung fortlaufend berechnet wird. Die verbesserte Ausführungsform erweist sich dann als besonders vorteilhaft, wenn sich eine der hier relevanten Größen, beispielsweise die Substitutionsrate oder Ultrafiltrationsrate, aber auch der Hämatokrit des Bluts des Patienten während der Dialysebehandlung ändern. Auch kommt eine Neuberechnung des Längswiderstandes $R_b$ in Frage, wenn von Prae- auf Postdilution oder umgekehrt übergegangen werden sollte.

Eine weitere alternative Ausführungsform sieht eine Berechnung des Längswiderstands $R_b$ nicht nach Gleichung (7), sondern nach Gleichung (10) vor, die einen allgemeineren Polynom-Ansatz beschreibt. Es sind aber grundsätzlich auch andere Polynom-Ansätze möglich.

Eine besonders bevorzugte Ausführungsform sieht vor, dass nicht von einem konstanten Wert für den Hämatokrit H*kt* ausgegangen wird, der beispielsweise mittels einer Tastatur eingegeben oder nur einmal gemessen wird. Bei dieser Ausführungsform wird der Hämatokrit mit der Messeinheit 23 während der Blutbehandlung fortlaufend gemessen.

In der Figur ist die Datenleitung 23' zur Übertragung der Messwerte für den Hämatokrit in gestrichelter Linie dargestellt, da die Messung des Hämatokrits während der Blutbehandlung nicht zwingend erforderlich und nur bei der besonders bevorzugten Ausführungsform vorgesehen ist.

Während der Blutbehandlung wird darüber hinaus vorzugsweise fortlaufend oder zumindest zu verschiedenen Zeitpunkten mittels der Drucksensoren 20, 21 und 22 der Druck $P_{b,out}$ am blutseitigen Auslass, der Druck $P_{d,in}$ am dialysatseitigen Einlass und der Druck $P_{d,out}$ am dialysatseitigen Auslass gemessen. Die Recheneinheit 19, die die Messwerte für die Drücke über die Datenleitung 20', 21', 22' empfängt, berechnet aus den Drücken nach Gleichung (3) nun den unkorrigierten Transmembrandruck $P_{TM3}$. Als weitere Größe empfängt die Recheneinheit 19 von der Steuer- und Recheneinheit 18 die Blutflussrate $Q_b$, die vom Bediener vorgegeben werden kann. Aus der Blutflussrate $Q_b$, dem berechneten Längswiderstand $R_b$ des Dialysators und dem unkorrigierten Transmembrandruck $P_{TM3}$ berechnet die Recheneinheit 19 dann nach Gleichung (6) den korrigierten Wert für den Transmembrandruck $P_{TM}$ = TMP.

Der korrigierte Transmembrandruck TMP kann auf einer nicht dargestellten Anzeigeeinheit angezeigt und/oder für eine Steuerung oder Regelung der Blutbehandlungsvorrichtung verwendet werden.

## Patentansprüche

1. Verfahren zur Bestimmung des Transmembrandrucks während einer extrakorporalen Blutbehandlung, bei der Blut mit einer bestimmten Blutflussrate über eine arterielle Blutzuführleitung (6) eines extrakorporalen Blutkreislaufs (5A) in den Einlass (3a )einer ersten Kammer (3) eines durch eine semi-permeable Membran (2) in die erste und eine zweite Kammer (3, 4) unterteilten Dialysators (1) strömt und über eine venöse Blutrückführleitung (7) aus dem Auslass (3b) der ersten Kammer (3) des Dialysators (1) strömt, und Dialysierflüssigkeit über eine Dialysierflüssigkeitszuführleitung (10) in den Einlass (4a) der zweiten Kammer (4) des Dialysators (1) strömt und über eine Dialysierflüssigkeitsabführleitung (11) aus dem Auslass (4b) der zweiten Kammer (4) des Dialysators (1) strömt, wobei dem Blut mit einer bestimmten Flussrate über die Membran (2) des Dialysators (1) Flüssigkeit entzogen wird,
   mit folgenden Verfahrensschritten:

   Messen des Drucks auf der Blutseite am Einlass oder Auslass der ersten Kammer des Dialysators und auf der Dialysierflüssigkeitsseite am Einlass oder Auslass der zweiten Kammer des Dialysators und
   <Berechnen des Transmembrandrucks auf der Grundlage des auf der Blutseite und Dialysierflüssigkeitsseite gemessenen Drucks,

   **dadurch gekennzeichnet,**
   **dass** eine mit der Viskosität des Bluts korrelierende Größe bestimmt wird,
   **dass** eine Korrekturgröße für den Transmembrandruck bestimmt wird, die von einer
   mit der Viskosität des Bluts korrelierenden Größe abhängig ist, wobei zur Bestimmung der Korrekturgröße für den Transmembrandruck eine für den Strömungswiderstand des Dialysators charakteristische Kenngröße bestimmt wird, die von der mit der Viskosität des Bluts korrelierenden Kenngröße abhängig ist, und
   **dass** der Transmembrandruck auf der Grundlage des auf der Blutseite und Dialysierflüssigkeitsseite gemessenen Drucks und der Korrekturgröße für den Transmembrandruck berechnet wird.

2. Verfahren nach Anspruch 1, **dadurch gekennzeichnet, dass** die mit der Viskosität des Bluts korrelierende Größe der Hämatokrit des Bluts ist.

3. Verfahren nach Anspruch 1 oder 2, **dadurch gekennzeichnet, dass** der Druck auf der Blutseite in der Blutabführleitung am Auslass der ersten Kammer des Dialysators und der Druck auf der Dialysierflüssigkeitsseite in der Dialysierflüssigkeitszuführleitung am Einlass der zweiten Kammer des Dialysators und in der Dialysierflüssigkeitsabführleitung am Auslass der zweiten Kammer des Dialysators gemessen wird.

4. Verfahren nach einem der Ansprüche 1 bis 3, **dadurch gekennzeichnet, dass** die für den Strömungswiderstand des Dialysators charakteristische Kenngröße auf der Grundlage der mit der Viskosität des Bluts korrelierenden Kenngröße und der Flussrate der über die Membran des Dialysators entzogenen Flüssigkeit bestimmt wird.

5. Verfahren nach Anspruch 4, **dadurch gekennzeichnet, dass** die für den Strömungswiderstand des Dialysators charakteristische Kenngröße nach dem folgenden Polynom-Ansatz mit den Parametern $\alpha_0$, $\alpha_1$, $\alpha_2$, $\alpha_3$, $\alpha_4$ berechnet wird:

$$R_b (Hkt, Q_{tm}) = a_0 + a_1 * Hkt + a_2 * \frac{Q_{tm}}{Q_{tm,max}} + a_3 * Hkt * \frac{Q_{tm}}{Q_{tm,max}} + a_4 * \left( Hkt * \frac{Q_{tm}}{Q_{tm,max}} \right)^4$$

$Hkt$ : Hämatokrit
$Q_{tm}$ : Flussrate über die Dialysator-Membran
$Q_{tm,max}$ : Maximale Flussrate über die Dialysator-Membran

6. Verfahren nach einem der Ansprüche 1 bis 5, **dadurch gekennzeichnet, dass** die Korrekturgröße auf der Grundlage des Produktes der für den Strömungswiderstand charakteristischen Kenngröße und der Blutflussrate bestimmt wird.

7. Verfahren nach einem der Ansprüche 3 bis 6, **dadurch gekennzeichnet, dass** der Transmembrandruck aus dem auf der Blutseite und auf der Dialysierflüssigkeitsseite gemessenen Druck und der Korrekturgröße nach der folgenden Gleichung berechnet wird:

$$P_{TM} = P_{TM3} + \frac{1}{2} * R_b * Q_b$$

mit:

$R_b$ Längswiderstand des Dialysators auf der Blutseite
$Q_b$ Blutfluss

und

$$P_{TM3} = P_{b,out} - \frac{P_{d,in} + P_{d,out}}{2}$$

8. Verfahren nach einem der Ansprüche 1 bis 7, **dadurch gekennzeichnet, dass** die mit der Viskosität korrelierende Größe während der Blutbehandlung fortlaufend gemessen wird.

9. Vorrichtung zur Bestimmung des Transmembrandrucks für eine Blutbehandlungsvorrichtung zur Durchführung einer extrakorporalen Blutbehandlung, bei der Blut mit einer bestimmten Blutflussrate über eine arterielle Blutzuführleitung (6) eines extrakorporalen Blutkreislaufs (5A) in den Einlass (3a) einer ersten Kammer (3) eines durch eine semipermeable Membran (2) in die erste und eine zweite Kammer (3, 4) unterteilten Dialysators (1) strömt und über eine venöse Blutrückführleitung (7) aus dem Auslass (3b) der ersten Kammer (3) des Dialysators (1) strömt, und Dialysierflüssigkeit über eine Dialysierflüssigkeitszuführleitung (10) in den Einlass (4a) der zweiten Kammer (4) des Dialysators (1) strömt und über eine Dialysierflüssigkeitsabführleitung (11) aus dem Auslass (4b) der zweiten Kammer (4) des Dialysators (1) strömt, wobei dem Blut mit einer bestimmten Flussrate über die Membran (2) des Dialysators (1) Flüssigkeit entzogen wird,
wobei die Vorrichtung zur Bestimmung des Transmembrandrucks aufweist:

Mittel (20, 21, 22) zum Messen des Drucks auf der Blutseite am Einlass oder Auslass der ersten Kammer des Dialysators und auf der Dialysierflüssigkeitsseite am Einlass oder Auslass der zweiten Kammer des Dialysators und
Mittel (19) zum Berechnen des Transmembrandrucks auf der Grundlage des auf der Blutseite und Dialysierflüssigkeitsseite gemessenen Drucks,

**dadurch gekennzeichnet,**
**dass** die Mittel (19) zum Berechnen des Transmembrandrucks derart ausgebildet sind, dass eine Korrekturgröße für den Transmembrandruck bestimmt wird, die von einer mit der Viskosität des Bluts korrelierenden Größe abhängig ist, wobei zur Bestimmung der Korrekturgröße für den Transmembrandruck eine für den Strömungswiderstand des Dialysators charakteristische Kenngröße bestimmt wird, die von der mit der Viskosität des Bluts korrelierenden Kenngröße abhängig ist, und

EP 2 231 226 B1

**dass** der Transmembrandruck auf der Grundlage des auf der Blutseite und Dialysierflüssigkeitsseite gemessenen Drucks und der Korrekturgröße für den Transmembrandruck berechnet wird,

10. Vorrichtung nach Anspruch 9, **dadurch gekennzeichnet, dass** die mit der Viskosität des Bluts korrelierende Größe der Hämatokrit des Bluts ist.

11. Vorrichtung nach Anspruch 9 oder 10, **dadurch gekennzeichnet, dass** die Mittel (20, 21, 22) zum Messen des Drucks auf der Blutseite und auf der Dialysierflüssigkeitsseite aufweisen:

> Mittel (20) zum Messen des Drucks in der Blutabführleitung am Auslass der ersten Kammer des Dialysators, Mittel (21) zum Messen des Drucks in der Dialysierflüssigkeitszuführleitung am Einlass der zweiten Kammer des Dialysators und Mittel (22) zum Messen des Drucks in der Dialysierflüssigkeitsabführleitung am Auslass der zweiten Kammer des Dialysators.

12. Vorrichtung nach einem der Ansprüche 9 bis 11, **dadurch gekennzeichnet, dass** die Mittel (19) zum Berechnen des Transmembrandrucks derart ausgebildet sind, dass die für den Strömungswiderstand des Dialysators charakteristische Kenngröße auf der Grundlage der mit der Viskosität des Bluts korrelierenden Kenngröße und der Flussrate der über die Membran des Dialysators entzogenen Flüssigkeit bestimmt wird.

13. Vorrichtung nach Anspruch 12, **dadurch gekennzeichnet, dass** die Mittel (19) zum Berechnen des Transmembrandrucks derart ausgebildet sind, dass die für den Strömungswiderstand des Dialysators charakteristische Kenngröße nach dem folgenden Polynom-Ansatz mit den Parametern $\alpha_0$, $\alpha_1$, $\alpha_2$, $\alpha_3$, $\alpha_4$ berechnet wird:

$$R_b\left(Hkt, Q_{tm}\right) = a_0 + a_1 * Hkt + a_2 * \frac{Q_{tm}}{Q_{tm,max}} + a_3 * Hkt * \frac{Q_{tm}}{Q_{tm,max}} + a_4 * \left(Hkt * \frac{Q_{tm}}{Q_{tm,max}}\right)^4$$

$Hkt$ : Hämatokrit
$Q_{tm}$ : Flussrate über die Dialysator-Membran
$Q_{tm,max}$ : Maximale Flussrate über die Dialysator-Membran

14. Vorrichtung nach einem der Ansprüche 9 bis 13, **dadurch gekennzeichnet, dass** die Mittel (19) zum Berechnen des Transmembrandrucks derart ausgebildet sind, dass die Korrekturgröße auf der Grundlage des Produktes der für den Strömungswiderstand charakteristischen Kenngröße und der Blutflussrate bestimmt wird.

15. Vorrichtung nach einem der Ansprüche 11 bis 14 **dadurch gekennzeichnet, dass** die Mittel (19) zum Berechnen des Transmembrandrucks derart ausgebildet sind, dass der Transmembrandruck aus dem auf der Blutseite und der Dialysierflüssigkeitsseite gemessenen Drucks und der Korrekturgröße nach der folgenden Gleichung berechnet wird:

$$P_{TM} = P_{TM3} + \frac{1}{2} * R_b * Q_b$$

mit:

> $R_b$ Längswiderstand des Dialysators auf der Blutseite
> $Q_b$ Blutfluss

und

$$P_{TM3} = P_{b,out} - \frac{P_{d,in} + P_{d,out}}{2}$$

16. Vorrichtung nach einem der Ansprüche 9 bis 15, **dadurch gekennzeichnet, dass** die Vorrichtung zur Bestimmung des Transmembrandrucks Mittel (23) zum Messen der mit der Viskosität des Bluts korrelierenden Größe aufweist, wobei die Mittel (19) zum Berechnen des Transmembrandrucks derart ausgebildet sind, dass die während der Blutbehandlung fortlaufend gemessene Größe, die mit der Viskosität korreliert, der Berechnung des Transmembrandrucks zugrunde gelegt wird.

17. Vorrichtung zur extrakorporalen Blutbehandlung mit einer Vorrichtung nach einem der Anspruch 9 bis 16.

**Claims**

1. Method for determining the transmembrane pressure during extracorporeal blood treatment, in which blood flows, at a particular blood flow rate, via an arterial blood supply line (6) of an extracorporeal blood circuit (5A) into the inlet (3a) of a first chamber (3) of a dialyser (1) which is divided into the first and a second chamber (3, 4) by a semipermeable membrane (2), and flows out of the outlet (3b) of the first chamber (3) of the dialyser (1) via a venous blood return line (7), and in which dialysate flows into the inlet (4a) of the second chamber (4) of the dialyser (1) via a dialysate supply line (10) and flows out of the outlet (4b) of the second chamber (4) of the dialyser (1) via a dialysate removal line (11), fluid being removed from the blood at a particular flow rate via the membrane (2) of the dialyser (1), comprising the following method steps:

   measuring the pressure on the blood side at the inlet or outlet of the first chamber of the dialyser and on the dialysate side at the inlet or outlet of the second chamber of the dialyser, and
   calculating the transmembrane pressure on the basis of the pressure measured on the blood side and on the dialysate side,

   **characterised in that**
   a variable is determined which correlates with the viscosity of the blood,
   **in that** a correction value for the transmembrane pressure is determined which is dependent on a variable which correlates with the viscosity of the blood, a characteristic parameter for the flow resistance of the dialyser being determined in order to determine the correction value for the transmembrane pressure, which characteristic parameter is dependent on the parameter which correlates with the viscosity of the blood, and
   **in that** the transmembrane pressure is calculated on the basis of the pressure measured on the blood side and on the dialysate side and on the basis of the correction value for the transmembrane pressure.

2. Method according to claim 1, **characterised in that** the variable which correlates with the viscosity of the blood is the haematocrit of the blood.

3. Method according to either claim 1 or claim 2, **characterised in that** the pressure on the blood side is measured in the blood removal line at the outlet of the first chamber of the dialyser and the pressure on the dialysate side is measured in the dialysate supply line at the inlet of the second chamber of the dialyser and in the dialysate removal line at the outlet of the second chamber of the dialyser.

4. Method according to any of claims 1 to 3, **characterised in that** the characteristic parameter for the flow resistance of the dialyser is determined on the basis of the parameter which correlates with the viscosity of the blood and on the basis of the flow rate of the fluid which is removed via the membrane of the dialyser.

5. Method according to claim 4, **characterised in that** the characteristic parameter for the flow resistance of the dialyser is calculated according to the following polynomial formulation having the parameters $\alpha_0$, $\alpha_1$, $\alpha_2$, $\alpha_3$, $\alpha_4$:

$$R_b\left(Hkt, Q_{tm}\right) = a_0 + a_1 * Hkt + a_2 * \frac{Q_{tm}}{Q_{tm,max}} + a_3 * Hkt * \frac{Q_{tm}}{Q_{tm,max}} + a_4 * \left(Hkt * \frac{Q_{tm}}{Q_{tm,max}}\right)^4$$

   H$kt$ : haematocrit
   $Q_{tm}$ : flow rate via the dialyser membrane
   $Q_{tm,max}$ : maximum flow rate via the dialyser membrane

6. Method according to any of claims 1 to 5, **characterised in that** the correction value is determined on the basis of the product of the characteristic parameter for the flow resistance and the blood flow rate.

7. Method according to any of claims 3 to 6, **characterised in that** the transmembrane pressure is calculated from the pressure measured on the blood side and on the

$$P_{TM} = P_{TM3} + \frac{1}{2} * R_b * Q_b$$

dialysate side and from the correction value according to the following equation: where:

$R_b$ is the longitudinal resistance of the dialyser on the blood side
$Q_b$ is the blood flow

and

$$P_{TM3} = P_{b,out} - \frac{P_{d,in} + P_{d,out}}{2}$$

8. Method according to any of claims 1 to 7, **characterised in that** the variable which correlates with the viscosity is continuously measured during the blood treatment.

9. Device for determining the transmembrane pressure for a blood treatment device for carrying out an extracorporeal blood treatment, in which device blood flows, at a particular blood flow rate, via an arterial blood supply line (6) of an extracorporeal blood circuit (5A) into the inlet (3a) of a first chamber (3) of a dialyser (1) which is divided into the first and a second chamber (3, 4) by a semipermeable membrane (2), and flows out of the outlet (3b) of the first chamber (3) of the dialyser (1) via a venous blood return line (7), and in which dialysate flows into the inlet (4a) of the second chamber (4) of the dialyser (1) via a dialysate supply line (10) and flows out of the outlet (4b) of the second chamber (4) of the dialyser (1) via a dialysate removal line (11), fluid being removed from the blood at a particular flow rate via the membrane (2) of the dialyser (1),
the device for determining the transmembrane pressure comprising:

means (20, 21, 22) for measuring the pressure on the blood side at the inlet or outlet of the first chamber of the dialyser and on the dialysate side at the inlet or outlet of the second chamber of the dialyser, and
means (19) for calculating the transmembrane pressure on the basis of the pressure measured on the blood side and on the dialysate side,

**characterised in that**
the means (19) for calculating the transmembrane pressure are designed such that a correction value for the transmembrane pressure is determined which is dependent on the variable which correlates with the viscosity of the blood, a characteristic parameter for the flow resistance of the dialyser being determined in order to determine the correction value for the transmembrane pressure, which characteristic parameter is dependent on the variable which correlates with the viscosity of the blood, and
**in that** the transmembrane pressure is calculated on the basis of the pressure measured at the blood side and at the dialysate side and on the basis of the correction value for the transmembrane pressure.

10. Device according to claim 9, **characterised in that** the variable which correlates with the viscosity of the blood is the haematocrit of the blood.

11. Device according to either claim 9 or claim 10, **characterised in that** the means (20, 21, 22) for measuring the pressure on the blood side and on the dialysate side comprise:

means (20) for measuring the pressure in the blood removal line at the outlet of the first chamber of the dialyser, means (21) for measuring the pressure in the dialysate supply line at the inlet of the second chamber of the dialyser and means (22) for measuring the pressure in the dialysate removal line at the outlet of the second chamber of the dialyser.

12. Device according to any of claims 9 to 11, **characterised in that** the means (19) for calculating the transmembrane

13

pressure are designed such that the characteristic parameter for the flow resistance of the dialyser is determined on the basis of both the parameter which correlates with the viscosity of the blood and the flow rate of the fluid which is removed via the membrane of the dialyser.

**13.** Device according to claim 12, **characterised in that** the means (19) for calculating the transmembrane pressure are designed such that the characteristic parameter for the flow resistance of the dialyser is calculated according to the following polynomial

$$R_b \, (Hkt, Q_{tm}) = a_0 + a_1 * Hkt + a_2 * \frac{Q_{tm}}{Q_{tm,max}} + a_3 * Hkt * \frac{Q_{tm}}{Q_{tm,max}} + a_4 * \left( Hkt * \frac{Q_{tm}}{Q_{tm,max}} \right)^4$$

formulation having the parameters $\alpha_1$, $\alpha_2$, $\alpha_3$, $\alpha_4$:

H$kt$ : haematocrit
$Q_{tm}$ : flow rate via the dialyser membrane
$Q_{tm,\,max}$ : maximum flow rate via the dialyser membrane

**14.** Device according to any of claims 9 to 13, **characterised in that** the means (19) for calculating the transmembrane pressure are designed such that the correction value is determined on the basis of the product of the characteristic parameter for the flow resistance and the blood flow rate.

**15.** Device according to any of claims 11 to 14, **characterised in that** the means (19) for calculating the transmembrane pressure are designed such that the transmembrane pressure is determined from the pressure measured on the blood side and on the dialysate side and from the correction value according to the following equation:

$$P_{TM} = P_{TM3} + \frac{1}{2} * R_b * Q_b$$

where:

$R_b$ is the longitudinal resistance of the dialyser on the blood side
$Q_b$ is the blood flow

and

$$P_{TM3} = P_{b,out} - \frac{P_{d,in} + P_{d,out}}{2}$$

**16.** Device according to any of claims 9 to 15, **characterised in that** the device for determining the transmembrane pressure comprises means (23) for measuring the variable which correlates with the viscosity of the blood, the means (19) for calculating the transmembrane pressure being designed such that the variable which is measured continuously during the blood treatment and correlates with the viscosity is based on the calculation of the transmembrane pressure.

**17.** Device for extracorporeal blood treatment, comprising a device according to any of claims 9 to 16.


**Revendications**

**1.** Procédé servant à déterminer la pression transmembranaire au cours d'un traitement du sang extracorporel, dans lequel du sang s'écoule, à un débit sanguin déterminé, par l'intermédiaire d'une conduite d'amenée de sang (6) artérielle d'un circuit sanguin extracorporel (5A), dans l'entrée (3a) d'un premier compartiment (3) d'un dialyseur (1) divisé par une membrane (2) semi-perméable en le premier compartiment et en un deuxième compartiment (3, 4) et s'écoule, par l'intermédiaire d'une conduite de reflux de sang (7) veineuse, de la sortie (3b) du premier compartiment (3) du dialyseur (1), et du liquide de dialyse s'écoule, par l'intermédiaire d'une conduite d'amenée de liquide de dialyse (10), dans l'entrée (4a) du deuxième compartiment (4) du dialyseur (1) et s'écoule, par l'intermédiaire de la conduite d'évacuation de liquide de dialyse (11), de la sortie (4b) du deuxième compartiment (4) du dialyseur (1), du liquide étant prélevé du sang à un débit déterminé par l'intermédiaire de la membrane (2) du dialyseur (1),

comprenant des étapes de procédé qui suivent :

la mesure de la pression du côté du sang à l'entrée ou à la sortie du premier compartiment du dialyseur et du côté du liquide de dialyse à l'entrée ou à la sortie du deuxième compartiment du dialyseur, et

le calcul de la pression transmembranaire sur la base de la pression mesurée du côté du sang et du côté du liquide de dialyse,

**caractérisé en ce**

**qu'**une grandeur en corrélation avec la viscosité du sang est déterminée,

en ce qu'une grandeur de correction est déterminée pour la pression transmembranaire, laquelle dépend d'une grandeur en corrélation avec la viscosité du sang, une grandeur caractéristique typique pour la résistance à l'écoulement du dialyseur étant déterminée afin de déterminer la grandeur de correction pour la pression transmembranaire, laquelle dépend de la grandeur caractéristique en corrélation avec la viscosité du sang, et

en ce que la pression transmembranaire est calculée sur la base de la pression mesurée du côté du sang et du côté du liquide de dialyse et sur la base de la grandeur de correction pour la pression transmembranaire.

2. Procédé selon la revendication 1, **caractérisé en ce que** la grandeur en corrélation avec la viscosité du sang est l'hématocrite du sang.

3. Procédé selon la revendication 1 ou 2, **caractérisé en ce que** la pression du côté du sang est mesurée dans la conduite d'évacuation de sang à la sortie du premier compartiment du dialyseur, et **en ce que** la pression du côté du liquide de dialyse est mesurée dans la conduite d'amenée de liquide de dialyse à l'entrée du deuxième compartiment du dialyseur et dans la conduite d'évacuation de liquide de dialyse à la sortie du deuxième compartiment du dialyseur.

4. Procédé selon l'une quelconque des revendications 1 à 3, **caractérisé en ce que** la grandeur caractéristique typique pour la résistance à l'écoulement du dialyseur est déterminée sur la base de la grandeur caractéristique en corrélation avec la viscosité du sang et sur la base du débit du liquide prélevé par l'intermédiaire de la membrane du dialyseur.

5. Procédé selon la revendication 4, **caractérisé en ce que** la grandeur caractéristique typique pour la résistance à l'écoulement du dialyseur est calculée selon l'approche polynomiale qui suit avec les paramètres $\alpha_0, \alpha_1, \alpha_2, \alpha_3, \alpha_4$ :

$$R_b(Hkt, Q_{tm}) = a_0 + a_1 * Hkt + a_2 * \frac{Q_{tm}}{Q_{tm,max}} + a_3 * Hkt * \frac{Q_{tm}}{Q_{tm,max}} + a_4 * \left( Hkt * \frac{Q_{tm}}{Q_{tm,max}} \right)^4$$

où

H$kt$ désigne l'hématocrite,

$Qtm$ désigne le débit par la membrane du dialyseur

$Qtm$, $max$ désigne le débit maximal par la membrane du dialyseur.

6. Procédé selon l'une quelconque des revendications 1 à 5, **caractérisé en ce que** la grandeur de correction est déterminée sur la base du produit de la grandeur caractéristique typique pour la résistance à l'écoulement et du débit sanguin.

7. Procédé selon l'une quelconque des revendications 3 à 6, **caractérisé en ce que** la pression transmembranaire est calculée à partir de la pression mesurée du côté du sang et du côté du liquide de dialyse et à partir de la grandeur de correction selon l'équation qui suit :

$$P_{TM} = P_{TM3} + \frac{1}{2} * R_b * Q_b$$

où

> $R_b$ désigne la résistance longitudinale du dialyseur du côté du sang,
> $Q_b$ désigne le débit sanguin,

et

$$P_{TM3} = P_{b,out} - \frac{P_{d,in} + P_{d,out}}{2}$$

8. Procédé selon l'une quelconque des revendications 1 à 7, **caractérisé en ce que** la grandeur en corrélation avec la viscosité est mesurée en continu au cours du traitement du sang.

9. Dispositif servant à déterminer la pression transmembranaire pour un dispositif de traitement du sang, servant à mettre en oeuvre un traitement du sang extracorporel, dans lequel du sang s'écoule, à un débit sanguin déterminé, par l'intermédiaire d'une conduite d'amenée de sang (6) artérielle d'un circuit sanguin extracorporel (5A), dans l'entrée (3a) d'un premier compartiment (3) d'un dialyseur (1) divisé par une membrane (2) semi-perméable en le premier et en un deuxième compartiment (3,4) et s'écoule, par l'intermédiaire d'une conduite de reflux de sang (7) veineuse, de la sortie (3b) du premier compartiment (3) du dialyseur (1), et du liquide de dialyse s'écoule, par l'intermédiaire d'une conduite d'amenée de liquide de dialyse (10), dans l'entrée (4a) du deuxième compartiment (4) du dialyseur (1) et s'écoule, par l'intermédiaire d'une conduite d'évacuation de liquide de dialyse (11), de la sortie (4b) du deuxième compartiment (4) du dialyseur (1), du liquide étant prélevé du sang à un débit déterminé par l'intermédiaire de la membrane (2) du dialyseur (1),
le dispositif servant à déterminer la pression transmembranaire présentant :

> des moyens (20, 21, 22) servant à mesurer la pression du côté du sang à l'entrée ou à la sortie du premier compartiment du dialyseur et du côté du liquide de dialyse à l'entrée ou à la sortie du deuxième compartiment du dialyseur, et
> des moyens (19) servant à calculer la pression transmembranaire sur la base de la pression mesurée du côté du sang et du côté du liquide de dialyse,

**caractérisé en ce que** les moyens (19) servant à calculer la pression transmembranaire sont réalisés de telle manière qu'une grandeur de correction est déterminée pour la pression transmembranaire, laquelle dépend de la grandeur en corrélation avec la viscosité du sang, une grandeur caractéristique typique pour la résistance à l'écoulement du dialyseur étant déterminée afin de déterminer la grandeur de correction pour la pression transmembranaire, laquelle dépend de la grandeur caractéristique en corrélation avec la viscosité du sang, et
en ce que la pression transmembranaire est calculée sur la base de la pression mesurée du côté du sang et du côté du liquide de dialyse et sur la base de la grandeur de correction pour la pression transmembranaire.

10. Dispositif selon la revendication 9, **caractérisé en ce que** la grandeur en corrélation avec la viscosité du sang est l'hématocrite du sang.

11. Dispositif selon la revendication 9 ou 10, **caractérisé en ce que** les moyens (20, 21, 22) servant à mesure la pression du côté du sang et du côté du liquide de dialyse présentent :

> des moyens (20) servant à mesurer la pression dans la conduite d'évacuation de sang à la sortie du premier compartiment du dialyseur,
> des moyens (21) servant à mesurer la pression dans la conduite d'amenée de liquide de dialyse à l'entrée du deuxième compartiment du dialyseur et des moyens (22) servant à mesurer la pression dans la conduite d'évacuation de liquide de dialyse à la sortie du deuxième compartiment du dialyseur.

**12.** Dispositif selon l'une quelconque des revendications 9 à 11, **caractérisé en ce que** les moyens (19) servant à calculer la pression transmembranaire sont réalisés de telle manière que la grandeur caractéristique typique pour la résistance à l'écoulement du dialyseur est déterminée sur la base de la grandeur caractéristique en corrélation avec la viscosité du sang et sur la base du débit du liquide prélevé par l'intermédiaire de la membrane du dialyseur.

**13.** Dispositif selon la revendication 12, **caractérisé en ce que** les moyens (19) servant à calculer la pression transmembranaire sont réalisés de telle manière que la grandeur caractéristique typique pour la résistance à l'écoulement du dialyseur est calculée selon l'approche polynomiale qui suit avec les paramètres $\alpha_0$, $\alpha_1$, $\alpha_2$, $\alpha_3$, $\alpha_4$ :

$$R_b(Hkt, Q_{tm}) = a_0 + a_1 * Hkt + a_2 * \frac{Q_{tm}}{Q_{tm,max}} + a_3 * Hkt * \frac{Q_{tm}}{Q_{tm,max}} + a_4 * \left( Hkt * \frac{Q_{tm}}{Q_{tm,max}} \right)^4$$

où

$Hkt$ désigne l'hématocrite,

Qtm désigne le débit par la membrane du dialyseur

$Qtm$, $max$ désigne le débit maximal par la membrane du dialyseur.

**14.** Dispositif selon l'une quelconque des revendications 9 à 13, **caractérisé en ce que** les moyens (19) servant à calculer la pression transmembranaire sont réalisés de telle manière que la grandeur de correction est déterminée sur la base du produit de la grandeur caractéristique typique pour la résistance à l'écoulement et du débit sanguin.

**15.** Dispositif selon l'une quelconque des revendications 11 à 14, **caractérisé en ce que** les moyens (19) servant à calculer la pression transmembranaire sont réalisés de telle manière que la pression transmembranaire est calculée à partir de la pression mesurée du côté du sang et du côté du liquide de dialyse et à partir de la grandeur de correction selon l'équation qui suit :

$$P_{TM} = P_{TM3} + \frac{1}{2} * R_b * Q_b$$

où

$R_b$ désigne la résistance longitudinale du dialyseur du côté du sang,

$Q_b$ désigne le débit sanguin,

et

$$P_{TM3} = P_{b,out} - \frac{P_{d,in} + P_{d,out}}{2}$$

**16.** Dispositif selon l'une quelconque des revendications 9 à 15, **caractérisé en ce que** le dispositif servant à déterminer la pression transmembranaire présente des moyens (23) servant à mesurer la grandeur en corrélation avec la viscosité du sang, les moyens (19) servant à calculer la pression transmembranaire étant réalisés de telle manière que la grandeur mesurée en continu au cours du traitement du sang, laquelle est corrélée à la viscosité, est sous-jacente au calcul de la pression transmembranaire.

**17.** Dispositif servant au traitement du sang extracorporel comprenant un dispositif selon l'une quelconque des revendications 9 à 16.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0212127 A
- WO 03028860 A1
- US 20040186410 A1